# EUROPÄISCHE PATENTANMELDUNG

(11) **EP 4 542 224 A1**
(43) Veröffentlichungstag der Anmeldung: **23.04.2025**
(21) Anmeldenummer: 24206781.7
(22) Anmeldetag: 15.10.2024
(51) Int. Cl.: G01N 33/38, G01M 5/00, G01N 27/20, G01N 33/00

(54) **RISSERKENNUNGSEINRICHTUNG UND OBJEKT MIT ZUMINDEST EINER SOLCHEN**

(30) Priorität: 20.10.2023 DE 102023128975
(71) Anmelder: Binder, Erhard, 51643 Gummersbach (DE)
(72) Erfinder:
(74) Vertreter: Rebbereh, Cornelia

(57) **Zusammenfassung**

Bei einer Risserkennungseinrichtung (1) zur Überwachung eines Objekts (8) auf Rissbildung an der Objektoberfläche (80) überwacht die Risserkennungseinrichtung (1) vollautomatisch die Rissbildung auf der Objektoberfläche (80) des Objekts (8) und umfasst zumindest ein Trägerelement (2) mit zumindest einer Leiterbahnschleife (3) und/oder zumindest einer mäandrierten Leiterbahn (4) und/oder sich kreuzenden und gegeneinander isolierten Leiterbahnen (50, 51, 52, 53, 54, 55, 56, 59) mit an den Kreuzungspunkten (57) der sich kreuzenden und gegeneinander isolierten Leiterbahnen (50, 51, 52, 53, 54, 55, 56, 59) angeordneten und mit den Leiterbahnen (50, 51, 52, 53, 54, 55, 56, 59) verbundenen Dioden (58), zumindest eine Verbindungseinrichtung zum Verbinden des zumindest einen Trägerelements (2) mit der Objektoberfläche (80) und zumindest eine oder zumindest einen Teil einer Auswerteeinheit (6), wobei die zumindest eine Auswerteeinheit (6) mit den Enden (30, 31, 40, 41, 50a, 50b, 51a, 51b, 52a, 52b, 53a, 53b, 55a, 55b, 56a, 56b) der Leiterbahn (3, 4) oder Leiterbahnen (50, 51, 52, 53, 54, 55, 56, 59) verbunden ist und kontinuierlich die Leiterbahn(en) (3, 4, 50, 51, 52, 53, 54, 55, 56, 59) auf Unterbrechungen überprüft.

## Beschreibung

Die Erfindung betrifft eine Risserkennungseinrichtung zur vollautomatischen Überwachung eines Objekts auf Rissbildung, die an der Objektoberfläche auftritt oder sich aus dem Innern des Objekts zu dessen Objektoberfläche oder von der Innenseite einer Objektwandung zu deren Außenseite fortsetzt, sowie ein Objekt mit zumindest einer solchen Risserkennungseinrichtung.

Beton- und Stahlbauwerke sind von ihrer Konstruktion her auf eine Lebensdauer von mehreren Jahrzehnten ausgelegt. Bei Bauten aus den 1960er und 1970er Jahren ist teilweise heute bereits das Ende der geplanten Lebensdauer erreicht. Dies betrifft insbesondere Brückenbauwerke. Gerade bei diesen haben sich die Anforderungen an deren Belastbarkeit durch den zunehmenden Verkehr deutlich erhöht: Ferner sind Lastkraftwagen (Lkws) heutzutage schwerer als im letzten Jahrhundert, was insbesondere auch Brückenbauwerke deutlich mehr belastet. Aber auch Personenkraftwagen (Pkws) werden zunehmend schwerer aufgrund ihrer gegenüber früheren Zeiten zunehmenden Größe und des Zusatzgewichts von Batteriespeichern. Damit können nicht nur Brückenbauwerke, sondern beispielsweise auch ältere Parkhäuser an die Grenze ihrer Belastbarkeit gelangen. Die meisten älteren Parkhäuser sind nicht für die schweren modernen Fahrzeuge ausgelegt. Vor 20 Jahren wog ein Pkw noch ca. 1.000 kg, heutige Pkws wiegen oftmals doppelt so viel, also 2.000 kg und mehr.

Vorschriften für die Prüfung von Betonbauwerken sind in der DIN 1076 (1999) (Ingenieurbauwerke im Zuge von Straßen und Wegen - Überwachung und Prüfung) geregelt. Danach sind manuell und handnah durchzuführende Hauptprüfungen alle 6 Jahre vorgesehen und eine sogenannte Einfache Prüfung alle 3 Jahre. Dieses Intervall ist für ältere Bauwerke je nach Belastungsgrad viel zu lang. Daher besteht der Bedarf nach einer Überwachungseinrichtung, die größere Flächen bzw. Bauwerke überwachen kann und einfach, kostengünstig und kontinuierlich arbeitet und die nachträglich an vorhandenen Bauwerken angebracht werden kann. Die Überwachungseinrichtung soll in der Lage sein, sowohl vorhandene Risse auf Vergrößerung zu überwachen als auch das Auftreten neuer Risse von der Außenseite bzw. -fläche von stark belasteten Bereichen von Objekten in Form von Bauwerken aus zu erkennen.

Vorhandene Einrichtungen erfüllen diese Anforderungen nicht. Beispielsweise sind Gipsmarken bekannt, die an vorhandenen Rissen zwar einfach anzubringen sind, die jedoch manuell überwacht werden müssen. Entsprechendes gilt für einfache Messschieber, die als Rissmonitor verwendet und auf vorhandene Risse aufgebracht werden.

Aus der US 2023/0324321 A1 ist eine beschichtete Struktur mit einem Überwachungssystem bekannt, enthaltend eine Basis mit einer Basisoberfläche, ein Beschichtungssystem mit einer oder mehreren Schichten einer gehärteten Beschichtung, die einen Schutz gegen Oberflächenverschlechterung bietet und mit der Basisoberfläche in einer Basisgrenzfläche verbunden ist und sich in einer Dickenrichtung zu einer äußeren Beschichtungsoberfläche erstreckt, zumindest eine Elektrode aus einem leitfähigen Material, die in das Beschichtungssystem eingebettet ist. Das Überwachungssystem ist so konfiguriert, dass es ein Eingangssignal in der zumindest einen Elektrode erzeugt und ein Ausgangssignal aus der zumindest einen Elektrode und aus dem Ausgangssignal ausliest, um einen Bruch einer oder mehrerer Schichten der gehärteten Beschichtung zu erkennen.

Die DE 10 2021 112 608 A1 offenbart eine Vorrichtung zur Strukturüberwachung einer mit der Vorrichtung beaufschlagbaren Oberfläche, die eine Trägerschicht und ein Signalverarbeitungsmittel umfasst, wobei die Trägerschicht mit elektrischen Leiterbahnen beaufschlagt ist, das Signalverarbeitungsmittel zur Überwachung einer elektrischen Eigenschaft der Leiterbahnen mit diesen elektrisch verbunden ist, die mit den elektrischen Leiterbahnen beaufschlagte Trägerschicht geschlitzt ist, so dass die Trägerschicht öffenbare Einschnitte und die elektrischen Leiterbahnen darüber öffenbare Kontaktstellen aufweisen, so dass bei geschlossenen Einschnitten auch die Kontaktstellen geschlossen sind und bei geöffneten Einschnitten auch die Kontaktstellen geöffnet und damit die elektrischen Leiterbahnen reversibel unterbrochen sind. Die von dem Signalverarbeitungsmittel überwachte elektrische Eigenschaft der Leiterbahnen ist so gewählt, dass diese für den Fall geschlossener Kontaktstellen einen anderen Wert aufweist als bei geöffneten Kontaktstellen. Auf diese Weise soll eine Vorrichtung zur Strukturüberwachung bereitgestellt werden, die kostengünstig herstellbar, flexibel einsetzbar sowie wiederverwendbar ist.

Aus der DE 60 2006 000 700 T2 ist ein beschichteter Faserstoff bekannt, der einen textilen Kern umfasst, der auf zumindest einer seiner Seiten mit einer Beschichtung bedeckt ist, und eine durchgehende Bahn umfasst, die auf der Basis eines elektrisch leitenden Werkstoffs hergestellt ist, der auf die Beschichtung aufgetragen ist und einen zumindest eine Zone der Faserstoffs durchlaufenden elektrischen Widerstand bildet. Die Bahn bildet ein kontinuierliches Muster, das ineinander verschachtelte Wellen bildet.

Systeme zur Messung der Änderung der Breite von Rissen mit Datenübertragung per Mobiltelefonnetz sind ebenfalls bekannt. Allerdings stellen sie lediglich eine Lösung für vorhandene Risse dar und erlauben lediglich ein punktuelles Messen.

Ferner sind Dehnungsmessungen über Dehnungsmessstreifen, optische Faserelemente oder FBGs bekannt. Hierbei können Dehnungen allerdings nur punktuell und bei größeren Objekten nur in einer Dimension detektiert werden. Für eine flächendeckende Überwachung sind die Einrichtungen nicht vorgesehen und auch nicht geeignet. Diese bekannten Einrichtungen und Verfahren erfordern einen hohen technischen Aufwand und sind dementsprechend teuer.

Ebenfalls bekannt ist die Überwachung auf Rissbildung mit piezoelektrischen Schall-Sensoren. Dies kann passiv erfolgen durch das Abhören von z.B. durch die Belastung über Fahrzeuge erzeugte Schallschwingungen oder aktiv durch das gezielte Aufbringen von Schallsignalen, deren Fortpflanzung und Modulation über einen Sensor erfasst wird. Diese Einrichtungen und Verfahren sind noch in der Erprobung, liefern meist ungenaue und unsichere Ergebnisse, und sie sind in der Handhabung sehr aufwendig.

Der vorliegenden Erfindung liegt daher die Aufgabe zugrunde, eine Risserkennungseinrichtung zur Überwachung eines Objekts auf Rissbildung an der Objektoberfläche sowie ein Objekt mit zumindest einer solchen Risserkennungseinrichtung zu schaffen, die die vorstehenden Nachteile nicht aufweist, vielmehr größere Flächen von Objekten überwachen kann, dabei kostengünstig ausgebildet ist und kontinuierlich, wie in wiederkehrenden Intervallen, arbeitet, nachträglich an vorhandenen Objekten angebracht werden kann und sowohl vorhandene Risse auf Vergrößerung überwachen als auch das Auftreten neuer Risse an Objekten sicher erkennen kann.

Die Aufgabe wird für eine Risserkennungseinrichtung nach dem Oberbegriff des Anspruchs 1 dadurch gelöst, dass die Risserkennungseinrichtung vollautomatisch die Rissbildung und/oder Rissvergrößerung eines bereits bestehenden Risses auf der Objektoberfläche des Objekts überwacht und zumindest ein Trägerelement mit zumindest einer Leiterbahnschleife und/oder zumindest einer mäandrierten Leiterbahn und/oder sich kreuzenden und gegeneinander isolierten Leiterbahnen mit an den Kreuzungspunkten der sich kreuzenden und gegeneinander isolierten Leiterbahnen angeordneten und mit den Leiterbahnen verbundenen Dioden, zumindest eine Verbindungseinrichtung zum Verbinden des zumindest einen Trägerelements mit der Objektoberfläche und zumindest eine oder zumindest einen Teil einer Auswerteeinheit umfasst, wobei die zumindest eine Auswerteeinheit mit den Enden der Leiterbahn oder Leiterbahnen verbunden ist und kontinuierlich die Leiterbahn(en) auf Unterbrechungen überprüft. Die Aufgabe wird für ein Objekt mit zumindest einer solchen Risserkennungseinrichtung dadurch gelöst, dass das Objekt ein Bauwerk ist, insbesondere ein Beton- und/oder Stahlbauwerk, wie eine Brücke, eine Fahrzeugrampe, eine tragende Wand, eine Stellfläche in einem Parkhaus oder ein anderes tragendes Bauwerkselement, auf dessen Außenseite die Risserkennungseinrichtung aufgebracht ist. Die Aufgabe wird ferner für ein Objekt mit zumindest einer solchen Risserkennungseinrichtung dadurch gelöst, dass das Objekt eine Lkw-Plane eines Lastkraftwagens zur Abdeckung der Ladefläche des Lastkraftwagens ist, wobei die Risserkennungseinrichtung zum Erkennen einer Beschädigung der Plane dient, die Lkw-Plane eine Innenseite und eine Außenseite aufweist und die zumindest eine Risserkennungseinrichtung auf der Innenseite der Lkw-Plane angeordnet ist. Weiterbildungen der Erfindung sind in den abhängigen Ansprüchen definiert.

Dadurch werden eine Risserkennungseinrichtung und ein Objekt mit einer solchen geschaffen, wobei eine vollautomatische Überwachung des Objekts auf Rissbildung und/oder auf Rissvergrößerung eines bereits bestehenden Risses an der Objektoberfläche dadurch ermöglicht wird, dass einerseits zumindest ein Trägerelement mit zumindest einer Leiterbahnschleife und/oder zumindest einer mäandrierten Leiterbahn und/oder sich kreuzenden und gegeneinander isolierten Leiterbahnen, wobei an den Kreuzungspunkten die voneinander bzw. gegeneinander isolierten Leiterbahnen mit jeweils einer Diode verbunden sind, zum Anzeigen einer Rissbildung oder Rissvergrößerung, andererseits zumindest eine Verbindungseinrichtung, die zum Verbinden der Risserkennungseinrichtung mit dem Objekt dient, und ferner zumindest eine Auswerteeinheit vorgesehen sind, wobei die Auswerteeinheit vollständig oder nur zu einem Teil auf dem Trägerelement angeordnet sein kann. Zumindest der Teil der Auswerteeinheit, der auf dem zumindest einen Trägerelement angeordnet ist, ist mit den Enden der zumindest einen Leiterbahn verbunden, um kontinuierlich deren Unversehrtheit bzw. eine eventuell auftretende Unterbrechung überprüfen zu können. Wird eine Unterbrechung detektiert, liegt eine Rissbildung oder eine Rissvergrößerung eines vorhandenen Risses, auf den die Risserkennungseinrichtung aufgebracht ist, vor. Tritt ein Riss auf, ist keine elektrisch leitende Verbindung im Verlauf der zumindest einen Leiterbahn an der Rissstelle mehr gegeben. Die Rissbildung kann an der Objektoberfläche auftreten oder sich aus dem Innern des Objekts zu dessen Objektoberfläche oder von der Innenseite einer Objektwandung zu deren Außenseite fortsetzen. Aufgrund des Vorsehens lediglich des zumindest einen Trägerelements mit darauf angeordneter zumindest einer Leiterbahn, der zumindest einen Verbindungseinrichtung und der Auswerteeinheit ist die Risserkennungseinrichtung kostengünstig und vergleichsweise einfach und somit auch robust ausgestaltet und ermöglicht aufgrund der Auswerteeinheit eine kontinuierliche Überwachung auch aus der Ferne. Kontinuierlich bedeutet hier vorliegend in Bezug auf das Abtasten der Dioden an den Kreuzungspunkten der sich kreuzenden und gegeneinander isolierten Leiterbahnen ein Abtasten in vorgebbaren periodischen Intervallen, z.B. alle 60 Minuten. Unter einer vollautomatischen Überwachung wird hier eine durch die Risserkennungseinrichtung selbständig und ohne Eingreifen von Bedienpersonen vorgenommene Überwachung eines Objekts verstanden. Unter einem Überwachen bzw. vollautomatischen Überwachen eines Objekts auf Rissbildung auf der Objektoberfläche wird vorliegend sowohl ein Überwachen auf das erstmalige Auftreten eines Risses, also eine Rissneubildung, als auch ein Überwachen auf eine Rissvergrößerung eines bereits bestehenden Risses, auf den die Risserkennungseinrichtung aufgebracht wird, verstanden. Unter einer Rissbildung wird vorliegend somit sowohl das erstmalige Auftreten eines Risses, somit eine Rissneubildung, als auch eine Vergrößerung eines bereits bestehenden Risses verstanden, auf den die Risserkennungseinrichtung aufgebracht ist. Die Risserkennungseinrichtung kann problemlos nachträglich an vorhandenen Objekten angebracht werden durch Verbinden der Risserkennungseinrichtung über deren zumindest eine Verbindungseinrichtung mit dem entsprechenden Objekt.

Ist das Objekt ein Bauwerk, eignet sich das Aufbringen der Risserkennungseinrichtung auf dessen Außenseite bzw. Außenfläche. Ist das Objekt eine Lkw-Plane, eignet sich das Aufbringen der Risserkennungseinrichtung auf deren zur Ladefläche des Lkws gerichteten Innenseite, um ein Aufschlitzen sicher erkennen zu können, jedoch für Personen von der Außenseite der Lkw-Plane aus nicht sichtbar zu sein.

Aus statischen Betrachtungen und Berechnungen sind die Bereiche eines Bauwerks bekannt, die am stärksten belastet sind. Die Risserkennungseinrichtung wird daher vorteilhaft gerade in diesen Bereichen an der Bauwerksoberfläche angebracht. Die Risserkennungseinrichtung umfasst weiter vorteilhaft zumindest eine Übertragungseinheit zum Übertragen der von der zumindest einen Auswerteeinheit ermittelten Daten zur Rissbildung und/oder Rissvergrößerung an eine zentrale Einheit. Damit können vorhandene Risse überwacht und neu entstehende Risse erkannt werden. Bei Einsatz eines Gitters aus sich kreuzenden und gegeneinander isolierten Leiterbahnen mit an den Kreuzungspunkten der sich kreuzenden und gegeneinander isolierten Leiterbahnen angeordneten und mit den Leiterbahnen verbundenen Dioden können ferner Risse in ihrer räumlichen Anordnung und Ausbreitung dokumentiert werden. Ein Riss kann bei seiner Ausbreitung ggf. mehrere Leiterbahnen durchtrennen, woraus sich seine räumliche Anordnung auf der Fläche des Gitters aus den sich kreuzenden und gegeneinander isolierten Leiterbahnen erkennen lässt. Aus diesen erfassten Daten bezüglich der Rissneubildung, Rissvergrößerung eines bestehenden Risses, auf den die Risserkennungseinrichtung aufgebracht ist, und der räumlichen Anordnung und Ausbreitung eines Risses kann ein Fachmann den Zustand des jeweiligen Bauwerks erkennen. Die Informationen zur Rissbildung und/oder Rissvergrößerung an der Oberfläche eines Bauwerks können über die zumindest eine Übertragungseinrichtung, z.B. über eine Funkverbindung, an eine zentrale Einheit übertragen werden, insbesondere können die Messdaten in Echtzeit übertragen werden. Die Auswerteeinheit selbst kann mit der zumindest einen Übertragungseinrichtung versehen sein zur kabellosen Übertragung der Messdaten, z.B. per Funk, so dass keine separate Übertragungseinheit erforderlich ist, oder die ermittelten Informationen bzw. Daten können beispielsweise über ein Bussystem kabelgebunden oder kabellos an eine Übertragungseinheit gesandt werden, über die diese Daten und Informationen an eine zentrale Einheit übertragen werden. Es ist somit eine Fernüberwachung des Objekts möglich, wobei vorteilhaft automatisch Zustandsmeldungen und auch Alarme an eine Fernüberwachungseinheit weitergegeben werden.

Insbesondere an der zentralen Einheit können die erfassten Messdaten des Messsystems in einer Datenbank abgelegt werden. Beispielsweise kann die Datenbank in einem Cloudspeicher abgelegt bzw. gespeichert werden oder sein. Es kann ein Anzeige- und Auswertesystem, wie ein Dashboard, vorgesehen werden, das die Anzeige des zeitlichen Verlaufs von Veränderungen der Messdaten in zeitlich komprimierter Form ermöglicht. Über eine solche Zeitkompression ist vorteilhaft eine Anzeige des Risswachstums zur weiteren Analyse des Schadensverlaufs möglich.

Die Ansprechschwelle der Risserkennungseinrichtung kann so ausgelegt sein, dass sie Risse ab einer Breite von 0,1 mm, insbesondere ab einer Breite von 0,2 mm, und/oder ein Risswachstum eines bestehenden Risses in einer Größenordnung von zumindest 0,1 mm, insbesondere zumindest 0,2 mm, verlässlich erkennt. Neu auftretende Risse ab einer Größe von ca. 0,1 mm können damit verlässlich erkannt und das Wachstum bereits vorhandener Risse bei einem Wachstum in gleicher Größenordnung sicher erfasst werden.

Die Risserkennungseinrichtung kann bei Versehen des zumindest einen Trägerelements mit einer mäandrierten Leiterbahn das Auftreten eines Risses im Bereich des auf das Objekt aufgebrachten, insbesondere aufgeklebten, Trägerelements feststellen. Bei Ausbilden der Leiterbahnschleife in Form eines schmalen Bandes kann eine Fläche in einer Dimension auf Risse überwacht werden. Bei Vorsehen eines aus den sich kreuzenden und gegeneinander isolierten Leiterbahnen gebildeten Gitters mit Dioden an den Kreuzungspunkten kann zusätzlich der Ort eines Risses ermittelt werden. Ist nur eine Leiterbahnschleife auf dem Trägerelement vorgesehen, kann die Risserkennungseinrichtung somit als schmales Band ausgebildet und auf das Bauwerk aufgebracht werden, um einen längeren Bereich eines Bauwerks in einer Dimension zu überwachen. Bei Vorsehen einer mäandrierten Leiterbahn sowie von sich kreuzenden und gegeneinander isolierten Leiterbahnen mit an den Kreuzungspunkten angeordneten Dioden werden eher flächige Formen der Risserkennungseinrichtung gebildet, die sogar sehr großflächig sein können. Das zumindest eine Trägerelement mit der zumindest einen darauf aufgebrachten, insbesondere auf dem Trägerelement aufgedruckten, Leiterbahn kann beispielsweise Abmessungen von bis 3,0 m x 15 m aufweisen, insbesondere Abmessungen von 0,4 m x 0,5 m bis 0,5 m x 1,5 m. Gerade Trägerelemente mit mäandrierter Leiterbahn können besonders große Abmessungen aufweisen, z.B. 3 m x 15 m. Es ist somit eine flächige Überwachung im Bereich des auf die Objektoberfläche aufgebrachten Trägerelements, das insbesondere in Form einer Trägerfolie ausgebildet sein kann, möglich. Insbesondere ist eine großflächige Überwachung über kaskadierte Trägerelemente bzw. Trägerfolien möglich. Hierbei sind mehrere Trägerelemente auf der Oberfläche des Objekts angeordnet und als Kaskade hintereinander geschaltet, so dass eine gezielte und eine besonders großflächige Überwachung der Objektoberfläche möglich ist.

Weiter vorteilhaft kann ein Rissereignis mit Zeit des Auftretens des Risses und Ort des Objekts, wie eines Bauwerks, an dem der Riss auftritt, und insbesondere der Position des Risses auf der Außenseite des Objekts und die Lage im Raum der Rissbildung oder der Risserweiterung bzw. Rissvergrößerung ermittelt bzw. erfasst werden. Dies ist, wie vorstehend bereits erwähnt, besonders gut bei Vorsehen sich kreuzender und gegeneinander isolierter Leiterbahnen mit an den Kreuzungspunkten angeordneten Dioden möglich, da bei diesem Aufbau der Sensorfläche, die durch die sich kreuzenden und gegeneinander isolierten Leiterbahnen mit den Dioden auf dem Trägerelement gebildet wird, eine genaue Zuordnung der Position und Lage eines Risses im Raum möglich ist.

Die Risserkennungseinrichtung ist weiter vorteilhaft mehrlagig aufgebaut und an die Anforderungen der jeweiligen Objektoberflächen angepasst: Beispielsweise bei einer grob verschalten Betonkonstruktion müssen, um eine ordnungsgemäße Messung durchführen zu können, Unebenheiten und kleinere Sprünge ausgeglichen werden. Daher erweist es sich als vorteilhaft, zumindest eine Ausgleichsschicht zum Ausgleich von Unebenheiten der Objektoberfläche vorzusehen. Insbesondere kann die zumindest eine Ausgleichsschicht die zumindest eine Verbindungseinrichtung umfassen. Es kann also insbesondere die Ausgleichsschicht aus einem solchen Material ausgebildet sein, dass einerseits ein Ausgleich von Unebenheiten und kleinen Versprüngen in der Objektoberfläche direkt ausgeglichen werden können, andererseits ein Befestigen der Risserkennungseinrichtung an der Objektoberfläche direkt ohne weitere Einrichtungen möglich ist. Die zumindest eine Ausgleichsschicht kann beispielsweise zumindest ein Schaummaterial mit haftender oder klebender Oberfläche umfassen oder aus einem solchen bestehen, also ein Schaumstoff mit klebender oder haftender Oberfläche sein, so dass die Ausgleichsschicht hierdurch mit der Verbindungseinrichtung versehen ist, da die klebende oder haftende Oberfläche ein Verbinden mit der Objektoberfläche ermöglicht und dementsprechend keine zusätzliche Verbindungseinrichtung vorgesehen zu werden braucht. Ferner ist es möglich, dass die zumindest eine Ausgleichsschicht aus zumindest einem Zwei-Komponenten-Material, insbesondere aus zumindest einem Zwei-Komponenten-Epoxidharz-Kleber oder einer aushärtenden Spachtelmasse, wie einem Epoxy-Mörtel, besteht/bestehen. Auch durch ein solches Zwei-Komponenten-Material ist einerseits ein Ausgleich von Unebenheiten und Versprüngen in der Objektoberfläche und andererseits ein Verbinden des Trägerelements mit der Objektoberfläche möglich.

Die Risserkennungseinrichtung wird häufig an der Außenseite der Objekte, wie von Bauwerken angebracht. Daher ist es wünschenswert, dass sie den Einflüssen von Wetter, Sonneneinstrahlung und starken Temperaturschwankungen widerstehen kann. Es erweist sich daher als sehr vorteilhaft, wenn die Risserkennungseinrichtung zumindest eine für UV-Strahlen zumindest wenig durchlässige UV-Schutzschicht aufweist. Vorteilhaft ist diese auf der der mit der Objektoberfläche zu verbindenden Seite der Risserkennungseinrichtung gegenüberliegenden Außenseite von dieser angeordnet, also auf der der Witterung ausgesetzten Außenseite der Risserkennungseinrichtung. Eine solche UV-Schutzschicht kann die darunterliegenden Schichten der Risserkennungseinrichtung gegen Verspröden und Altern durch starke Sonneneinstrahlung schützen. Anstelle einer UV-Schutzfolie als UV-Schutzschicht kann diese auch als Lackschicht ausgebildet und als Deckschicht und Wetterschutz aufgebracht werden, um die einzelnen Lagen der mehrlagigen Risserkennungseinrichtung vor Versprödung durch Sonnenlicht zu schützen.

Zum Schutz der an Kreuzungspunkten von sich kreuzenden und gegeneinander isolierten Leiterbahnen angeordneten Dioden vor mechanischer Beschädigung kann weiter vorteilhaft zumindest eine Abstandsschicht zwischen den Dioden angeordnet sein, deren Höhe zumindest der der Dioden entspricht. Es entsteht hierdurch eine im Wesentlichen glatte Oberfläche, die das Aufbringen der Risserfassungseinrichtung auf einem Objekt als Untergrund durch Anpressen mittels einer Rolle vereinfacht und auf die die zumindest eine Schutzschicht problemlos aufgebracht werden kann, z.B. blasenfrei über eine Rolle.

Das zumindest eine Trägerelement kann beispielsweise eine Kunststofffolie sein, die insbesondere aus Polycarbonat besteht. Auf diese kann die zumindest eine Leiterbahn besonders einfach z.B. aufgedruckt werden bzw. sein.

Das zumindest eine Trägerelement kann ferner einseitig aufgeraut sein, um eine gute Haftung der Ausgleichsschicht auf der Trägerelementunterseite zu ermöglichen.

Die Auswerteeinheit kann Elemente zur Selbstüberwachung enthalten, um zuverlässig einen Ausfall der Auswerteeinheit ganz oder in Teilen zu erkennen. An den Eingängen der Auswerteeinheit können z.B. Signalleitungen mit eindeutig definiertem Zustand, leitend und nicht-leitend, angeschlossen werden, um die Funktion der Eingangs-Ports der Auswerteeinheit zu testen. Über Schwingungen am Objekt, die z.B. durch regelmäßige Belastungen eines Brückenbauwerks oder eines anderen Bauwerks durch Verkehr erzeugt werden können, kann die zumindest eine Leiterbahn auf dem zumindest einen Trägerelement verspröden oder Mikrorisse an dieser auftreten. Die Auswerteeinheit kann daher vorteilhaft Algorithmen zur Signalüberwachung enthalten, um eine gelegentliche Kontaktunterbrechung durch Verspröden der zumindest einen Leiterbahn von einem echten Bruch aufgrund Auftretens eines Risses im Objekt, wie einem Bauwerk, unterscheiden zu können.

Die Risserkennungseinrichtung kann auf der Außenseite von Objekten, wie beispielsweise Außenflächen von gefährdeten Bauwerken, wie Stahlbrücken, Betonbrücken oder Rampen und Stellflächen von Parkhäusern aufgebracht werden oder an Objektflächen, die von Statikern oder Bauwerksprüfern als besonders gefährdet erkannt worden sind. Vorteilhaft wird die zumindest eine Risserkennungseinrichtung an einer Seite oder Unterseite des Bauwerks angeordnet, die nicht mechanisch belastet wird, also an einer Seite oder Unterseite eines Bauwerks angebracht, insbesondere aufgeklebt, an der keine mechanische Belastung, z.B. durch Autoverkehr, auftritt. Hierdurch kann die Risserkennungseinrichtung vor Beschädigung geschützt werden.

Lkw-Planen dienen zur Abdeckung der Ladefläche von Lkw-Anhängern oder zum Schutz des Laderaums von Lkws. Sattelauflieger, sog. Trailer, von Lkws werden an den Seiten oftmals durch Planen, die meist aus Folienmaterial bestehen, nach außen verschlossen. Die Abmessungen der jeweiligen Plane bzw. Folie bestimmt sich durch die Standard-Maße des Sattelaufliegers, die in Deutschland ca. 13,6 m x 2,7 m betragen. Im Stand des Lkws können diese Planen bzw. Folien leicht aufgeschlitzt werden, um Teile des Ladeguts zu entwenden. Dieses Problem tritt häufig an nicht oder nur wenig bewachten Parkplätzen auf, wie z.B. an Autobahnrastplätzen. Um die Plane bzw. Folie des Sattelaufliegers vor Aufschlitzen zu schützen kann die Risserkennungseinrichtung zum Erkennen von Rissen in Lkw-Planen verwendet werden. Durch diese kann ein Aufschlitzen der Lkw-Planen erkannt und entsprechend Alarm ausgelöst werden, um z.B. Diebstähle aus abgestellten Lkw-Trailern zu verhindern. Die Risserkennungseinrichtung kann daher vorteilhaft mit zumindest einer Einrichtung zum Ausgeben eines Alarms zusammenwirken zum Ausgeben eines Alarms bei Erkennen einer Beschädigung der Plane durch Aufschlitzen. Eine solche Einrichtung zum Ausgeben eines Alarms kann auch bereits auf dem zumindest einen Trägerelement der Risserkennungseinrichtung angeordnet werden.

Die Folie bzw. Lkw-Plane wird auf ihrer Innenseite mit der Risserkennungseinrichtung versehen. Die zumindest eine Leiterbahn kann insbesondere mäandrierend oder in ähnlicher Form angeordnet sein, so dass sie die gesamte Fläche der Lkw-Plane bedeckt und somit eine flächendeckende Überwachung möglich ist. Wird die Plane aufgeschnitten, so wird die Leiterbahn unterbrochen. Die Auswerteeinheit erkennt die Unterbrechung und kann ein Alarmsignal erzeugen oder einen Alarm auslösen und ein Signal an eine zentrale Überwachungseinheit des Sattelaufliegers oder des Lkws weiterleiten, von wo aus der Alarm weiterverarbeitet wird. Bei einem plötzlichen Anstieg des elektrischen Widerstandes aufgrund der durchtrennten Leiterbahn wird somit automatisch ein Alarmsignal ausgelöst. Dieses Alarmsignal kann direkt einen akustischen und/oder optischen Alarm auslösen oder an eine zentrale Überwachungseinheit des Lkws weiter geleitet werden.

Weiter vorteilhaft kann die Lkw-Plane als Trägerelement der Risserkennungseinrichtung ausgebildet und mit zumindest einer Leiterbahn versehen, insbesondere bedruckt, sein und/oder ein zusätzliches Trägerelement der Risserkennungseinrichtung vorgesehen und mit zumindest einer Leiterbahn versehen sein. Es kann somit eine bereits mit Trägerelement versehene Risserkennungseinrichtung auf die Innenseite der Lkw-Plane aufgebracht, insbesondere aufgeklebt werden. Alternativ ist es möglich, die Lkw-Plane selbst als Trägerelement der Risserkennungseinrichtung zu nutzen und die zumindest eine Leiterbahn sowie die zumindest eine Auswerteeinheit auf diese direkt aufzubringen. Die zumindest eine Leiterbahn kann vorzugsweise auf die Lkw-Plane direkt aufgedruckt werden. Das Vorsehen eines zusätzlichen Trägerelements kann dabei entfallen ebenso wie die zumindest eine Ausgleichsschicht. Die zumindest eine Verbindungseinrichtung ist bei Aufdrucken der zumindest einen Leiterbahn auf die Oberfläche der Lkw-Plane durch die haftende Eigenschaft des Druckmaterials zum Ausbilden der zumindest einen Leiterbahn auf der Lkw-Plane ausgebildet. Alle übrigen Schichten, die auf der zumindest einen Leiterbahn angeordnet werden können, können grundsätzlich auch weiterhin vorgesehen werden. Da die Risserkennungseinrichtung jedoch auf der Innenseite der Lkw-Plane angeordnet wird, kann z.B. die Schutzschicht zum Schutz der Oberfläche der darunter liegenden Schichten vor Versprödung und Altern durch UV-Licht entfallen, da diese Problematik an der Innenseite der Lkw-Plane eher selten zum Tragen kommt. Grundsätzlich kann daher die Risserkennungseinrichtung bei Anordnen der zumindest einen mäandrierten Leiterbahn direkt auf der Lkw-Plane auch lediglich die zumindest eine Leiterbahn und die zumindest eine Auswerteeinheit mit der Möglichkeit des Auslösens eines Alarms umfassen.

Zur näheren Erläuterung der Erfindung werden im Folgenden Ausführungsbeispiele von dieser näher anhand der Zeichnungen beschrieben. Diese zeigen in:
- Figur 1: eine Draufsicht auf eine erste Ausführungsform einer erfindungsgemäßen Risserkennungseinrichtung mit auf einem Trägerelement aufgebrachter mäandrierter Leiterbahn und Auswerteeinheit,
- Figur 2: eine Draufsicht auf eine zweite Ausführungsform einer erfindungsgemäßen Risserkennungseinrichtung mit auf einem Trägerelement aufgebrachter Leiterbahnschleife und Auswerteeinheit,
- Figur 3: eine Draufsicht auf eine dritte Ausführungsform einer erfindungsgemäßen Risserkennungseinrichtung mit auf einem Trägerelement aufgebrachtem Gitter aus sich kreuzenden und gegeneinander isolierten Leiterbahnen mit an den Kreuzungspunkten angeordneten Dioden und mit einer Auswerteeinheit,
- Figur 4: eine Schnittansicht einer weiteren Ausführungsform einer vierlagigen erfindungsgemäßen Risserkennungseinrichtung,
- Figur 5: eine Schnittansicht einer weiteren Ausführungsform einer achtlagigen erfindungsgemäßen Risserkennungseinrichtung als Prinzipskizze, und
- Figur 6: eine Schnittansicht einer weiteren Ausführungsform einer achtlagigen erfindungsgemäßen Risserkennungseinrichtung.

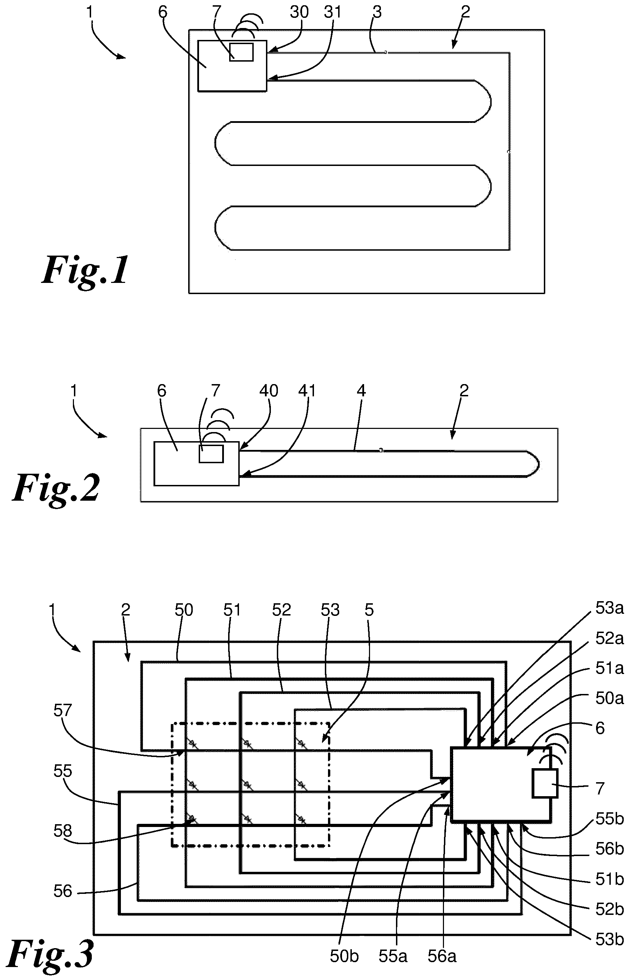

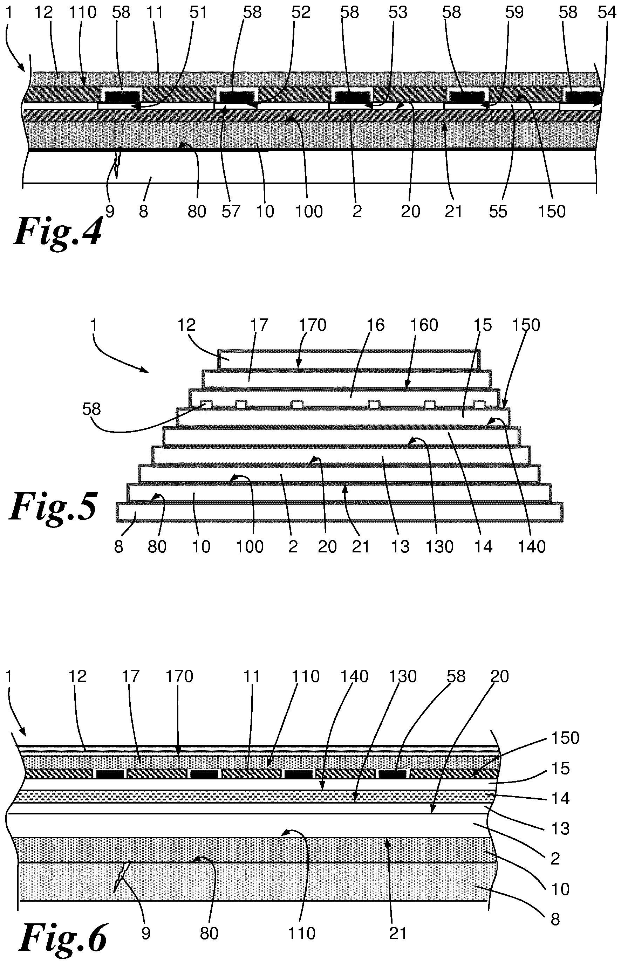

In den Figuren 1 bis 3 sind drei verschiedene Ausführungsvarianten einer Risserkennungseinrichtung 1 gezeigt, die zum Erkennen von sich in einem Objekt 8, wie beispielweise einem Bauwerk oder auch einer Lkw-Plane, ausbildenden Rissen 9 (siehe beispielhaft in den Figuren 4 und 6) oder in dieses durch Gewaltausübung darin eingebrachten Rissen 9, wie bei einem Aufschlitzen der Lkw-Plane, dient. In allen drei Ausführungsvarianten umfasst die Risserkennungseinrichtung 1 ein Trägerelement 2, auf das eine Leiterbahn bzw. mehrere Leiterbahnen aufgebracht sind. Insbesondere sind die Leiterbahnen auf das Trägerelement 2 aufgedruckt. Bei der Ausführungsvariante nach Figur 1 ist eine mäandrierte Leiterbahn 3 auf das Trägerelement 2 aufgebracht, insbesondere aufgedruckt. Bei der Ausführungsvariante nach Figur 2 ist eine Leiterbahnschleife 4 auf dem Trägerelement 2 angeordnet, insbesondere darauf aufgedruckt. Bei der Ausführungsform nach Figur 3 ist ein Gitter 5 mit sich kreuzenden und gegeneinander isolierten Leiterbahnen 50, 51, 52 und 53, 55, 56 auf dem Trägerelement 2 aufgebracht, insbesondere darauf aufgedruckt. An jedem der Kreuzungspunkte 57 der Leiterbahnen ist jeweils eine Diode 58 angeordnet, wie ebenfalls Figur 3 entnommen werden kann. Das Gitter 5 bzw. der Bereich der sich kreuzenden und gegeneinander isolierten Leiterbahnen mit den dort angeordneten Dioden 58 ist die Sensorfläche der Risserkennungseinrichtung 1.

Bei jeder der drei Ausführungsvarianten der Risserkennungseinrichtung 1 ist jeweils eine Auswerteeinheit 6 ebenfalls auf den Trägerelement 2 angeordnet und mit den jeweiligen Leiterbahnenden 30, 31 bzw. 40, 41 bzw. 50a, 50b, 51a, 51b, 52a, 52b, 53a, 53b, 55a, 55b, 56a, 56b elektrisch leitend verbunden, so dass eine Durchgängigkeit der jeweiligen Leiterbahn bzw. Leiterbahnschleife durch die Auswerteeinheit 6 erfasst werden kann. Wird eine zumindest eine Leiterbahn aufgrund der Rissbildung oder der Rissvergrößerung eines bestehenden Risses, auf den das Trägerelement der Risserkennungseinrichtung aufgebracht ist, durchtrennt, erhöht sich der elektrische Widerstand, so dass die Auswerteeinheit 6 die Rissbildung bzw. Rissvergrößerung hieraus erkennen kann.

Mit der Risserkennungseinrichtung 1 gemäß Figur 1 ist es möglich, nicht nur kleinflächige, sondern auch größere flächige Bereiche auf einem Objekt, wie einem Bauwerk oder eine Lkw-Plane, auf das Auftreten von Rissen 9 zu überwachen. Mit der Risserkennungseinrichtung 1 gemäß Figur 2, die bezüglich ihres Trägerelementes 2 als schmales Band ausgebildet ist, ist es besonders gut möglich, eine Fläche in einer Dimension auf auftretende Risse zu überwachen. Mit der Risserkennungseinrichtung 1 gemäß Figur 3 kann besonders gut der Ort und die Lage des jeweiligen Risses, somit dessen Position und Lage im Raum eindeutig ermittelt werden. Über das Gitter 5 und die jeweils an dessen Kreuzungspunkten 57 angeordneten Dioden 58 ist eine genaue Lagebestimmung eines auftretenden Risses 9 möglich.

Die Auswerteeinheiten 6 der Risserkennungseinrichtungen 1 gemäß Figur 1 bis 3 umfassen jeweils auch eine Übertragungseinheit 7. Mittels der Übertragungseinheit 7 ist insbesondere kabellos, z.B. per Funk, eine Übertragung der durch die Auswerteeinheit 6 ermittelten Daten zur Rissbildung und/oder Rissvergrößerung an eine zentrale Einheit möglich. Eine solche zentrale Einheit ist in den Figuren nicht gezeigt. Eine solche wird meist entfernt von dem jeweiligen auf Rissbildung bzw. Rissvergrößerung zu überwachenden Objekt angeordnet. In der zentralen Einheit bzw. an einem zentralen Ort können die erfassten Messdaten des Messsystems in Form der Risserkennungseinrichtung in einer Datenbank abgelegt und insbesondere ein Auswerte- und Anzeigesystem seitens des zentralen Einheit vorgesehen sein, wie beispielsweise ein Dashboard, das die Anzeige des zeitlichen Verlaufs der Veränderung der Messdaten, also einer Rissbildung, auch in zeitlich komprimierter Form ermöglicht. Gerade die Anzeige eines Risswachstums in einer solchen zeitlich komprimierten Form kann zur weiteren Analyse eines Schadensverlaufs dienen. Ist die Risserkennungseinrichtung 1 beispielsweise an einem Brückenbauwerk angeordnet, kann über den zeitlichen Verlauf des Risswachstums gezielt einerseits die Verkehrsführung über das Brückenbauwerk entsprechend angepasst, andererseits das Brückenbauwerk frühzeitig für den Verkehr gesperrt werden, um weitere Beschädigungen an diesen frühzeitig zu verhindern.

Anstelle einer kabellosen Übertragung der von der Auswerteeinheit 6 ermittelten auf eine Rissbildung bzw. Rissvergrößerung bezogener Messdaten über die auf der oder in der Auswerteeinheit 6 vorgesehenen Übertragungseinheit 7 kann auch ein Übermitteln der Messdaten und weiterer Informationen über ein Bussystem, das kabelgebunden oder kabellos mit der Auswerteeinheit 6 verbunden ist bzw. werden kann, vorgesehen werden. Über das Bussystem und ggf. eine weitere Übertragungseinheit kann ein Übertragen der Messdaten und weiterer Informationen an eine zentrale Einheit ermöglicht werden. Anstelle des Implementierens der Übertragungseinheit 7 bereits auf der Auswerteeinheit 6 kann auch eine separate Übertragungseinheit vorgesehen werden, die auf dem Objekt und/oder dem Trägerelement 2 der Risserkennungseinrichtung 1 angeordnet wird. Eine solche separate Übertragungseinheit kann kabellos oder kabelgebunden mit der Auswerteeinheit 6 verbunden werden bzw. kommunizieren und dementsprechend eine Übertragung der von der Auswerteeinheit 6 erfassten Messdaten an eine zentrale Einheit zur Weiterverarbeitung der Messdaten ermöglichen.

In den Figuren 4 bis 6 sind jeweils beispielhaft Schnittansichten der Risserkennungseinrichtung 1 gezeigt, wobei sich die Ausführungsformen der Risserkennungseinrichtung 1 gemäß Figur 4 bis 6 jeweils durch die Anzahl der Schichten der mehrlagigen Risserkennungseinrichtung 1 unterscheiden. Jede dieser drei Ausführungsvarianten enthält Dioden 58, wobei jeweils ein Schnitt durch das Gitter 5 bzw. die Sensorfläche mit den sich kreuzenden und gegeneinander isolierten Leiterbahnen 50, 51, 52, 53, 55, 56 und an den jeweiligen Kreuzungspunkten 57 von diesen angeordneten Dioden 58 gezeigt ist.

Bei der in Figur 4 gezeigten Ausführungsvariante der Risserkennungseinrichtung 1 ist auf der Objektoberfläche 80 des Objekt 8, das beispielsweise ein Beton- oder Stahlbauwerk sein kann, eine Ausgleichsschicht 10 zum Ausgleich von Unebenheiten der Objektoberfläche 80 angeordnet. Gerade bei grob verschalten Betonkonstruktionen weist deren Oberfläche Unebenheiten und kleinere Vorsprünge auf, die ein festes Anbringen der Risserkennungseinrichtung bzw. ein ordnungsgemäßes Arbeiten von dieser ggf. be- oder sogar verhindern können. Dementsprechend erweist es sich als vorteilhaft, die Ausgleichsschicht 10 auf die Objektoberfläche 80, also insbesondere Betonoberfläche, aufzubringen. Die Ausgleichsschicht 10 kann beispielsweise ein Schaummaterial mit klebender oder haftender Oberfläche oder eine aushärtende Spachtelmasse sein, die auf die Objektoberfläche 80 aufgebracht wird. Wenn die Ausgleichsschicht 10 als aushärtende Spachtelmasse, somit als Zwei-Komponenten-Material ausgebildet ist, haftet dieses selbstständig auf der Objektoberfläche 80, so dass keine zusätzliche Verbindungseinrichtung, die ein Befestigen der Risserkennungseinrichtung 1 auf der Objektoberfläche 80 ermöglicht, erforderlich ist. Insbesondere kann als Ausgleichschicht 10 ein Zwei-Komponenten-Epoxidharz-Kleber, der aus Kleber und Härter besteht, die vor dem Auftragen auf die Objektoberfläche 80 des Objekt 8 miteinander verrührt werden, verwendet werden. Bei Ausbilden der Ausgleichsschicht 10 aus Schaummaterial mit klebender Oberfläche bildet die klebende Oberfläche des Schaummaterials die erforderliche Verbindungseinrichtung zum Verbinden der Risserkennungseinrichtung 1 mit der Objektoberfläche 80 des Objekts 8. Die Ausgleichschicht 10 ist vorteilhaft eine Distanzschicht, die es ermöglicht, dass Risse ungefiltert weitergegeben werden, mit der jedoch zugleich Rauigkeiten und Höhenunterschiede gerade einer Betonoberfläche besonders effektiv ausgeglichen werden können.

Auf der Ausgleichsschichtoberfläche 100 ist in der in Figur 4 gezeigten Ausführungsvariante der Risserkennungseinrichtung 1 ein Trägerelement 2 angeordnet. Dies kann beispielsweise in Form einer Trägerfolie ausgebildet sein, insbesondere einer Trägerfolie aus Polycarbonat. Wie bereits in den Figuren 1 bis 3 gezeigt, dient das Trägerelement 2 zum Anordnen der Leiterbahnen. Diese können in jeglicher Form, beispielsweise in mäandrierter Form, wie in Figur 1 gezeigt, oder in Form von sich kreuzenden und als Gitter 5 bzw. Sensorfläche ausgebildeten Leiterbahnen auf dem Trägerelement 2 bzw. dessen Trägerelementoberfläche 20 angeordnet sein bzw. werden. Bei dem in Figur 4 gezeigten Ausführungsbeispiel ist die im Querschnitt gezeigte Leiterbahn 55 auf das Trägerelement 2 bzw. dessen Trägerelementoberfläche 20 aufgedruckt. Das Trägerelement 2 ist auf seiner Trägerelementunterseite 21 aufgeraut oder strukturiert, um ein besonders gutes Haften der Ausgleichsschicht 10 dort zu ermöglichen.

Angedeutet sind in Figur 4 ebenfalls die sich mit der Leiterbahn 55 kreuzenden Leiterbahnen 51, 52, 53, 54 und 59. Im Bereich der Kreuzungspunkte 57 der Leiterbahnen ist jeweils eine Diode 58 angeordnet. Die Dioden 58 können dementsprechend auf die jeweilige Oberfläche 150 der sich kreuzenden Leiterbahnen im Bereich der Kreuzungspunkte 57 aufgeklebt und/oder an die Leiterbahnen angelötet werden. In jedem Falle wird eine elektrisch leitende Verbindung zwischen Diode und der jeweiligen Leiterbahnoberfläche 150 vorgesehen, wobei die Leiterbahnen an ihren Kreuzungspunkten 57 gegeneinander isoliert sind. Eine elektrische Verbindung zwischen den Leiterbahnen erfolgt somit nur über die an den Kreuzungspunkten 57 angeordneten Dioden 58, die jeweils zwei sich kreuzende Leiterbahnen verbinden, somit mit beiden elektrisch leitend verbunden sind.

Um die Dioden 58 vor mechanischer Beschädigung zu schützen, ist bei der in Figur 4 gezeigten Ausführungsvariante der Risserkennungseinrichtung 1 zwischen diesen und neben diesen eine Abstandsschicht 11 angeordnet, deren Höhe zumindest der der Dioden 58 entspricht. Dies kann ebenfalls Figur 4 sehr gut entnommen werden. Durch Vorsehen der Abstandsschicht 11 werden die Dioden 58 vor einer mechanischen Beschädigung beim Anbringen der Risserkennungseinrichtung an dem Objekt 8, wie beispielsweise einem Bauwerk, geschützt. Ferner entsteht durch das Vorsehen der Abstandsschicht 11 eine im Wesentlichen ebene glatte Oberfläche, auf der eine weitere Schicht, nämlich eine für UV-Strahlen zumindest wenig durchlässige UV-Schutzschicht 12 aufgebracht werden kann. Durch Vorsehen der für UV-Strahlen im Wesentlichen undurchlässigen UV-Schutzschicht 12, die beispielsweise aus Folienmaterial bestehen oder als Lackschicht ausgebildet werden kann, werden die darunterliegenden Schichten der Risserkennungseinrichtung 1 gegen Verspröden und Altern durch starke Sonneneinstrahlung geschützt. Diese UV-Schutzschicht 12 kann beispielsweise auf die Abstandsschichtoberfläche 110 der Abstandsschicht 11 aufgeklebt werden. Durch das Vorsehen der Abstandsschicht 11, die für eine im Wesentlichen ebene und glatte Oberfläche sorgt, ist es beispielsweise möglich, mithilfe einer Rolle blasenfrei die UV-Schutzschicht 12 auf der Abstandsschichtoberfläche 110 der Abstandsschicht 11 aufzubringen. Ferner ermöglicht diese glatte Oberfläche das problemlose Aufbringen der Risserkennungseinrichtung 1 auf oder an dem Objekt 8 als Untergrund oder der weiteren Schichten auf der Ausgleichsschicht 10 durch Anpressen mittels einer Rolle.

Bei einer der beiden Ausführungsvarianten der Risserkennungseinrichtung 1 gemäß Figur 1 und Figur 2, bei denen keine sich kreuzenden Leiterbahnen und keine Dioden 58 an Kreuzungspunkten von sich kreuzenden Leiterbahnen angeordnet sind, wird üblicherweise die Abstandsschicht 11 nicht benötigt. Diese kann dementsprechend entfallen, so dass auf der jeweiligen Oberfläche 150 der mäandrierten Leiterbahn 3 bzw. Leiterbahnschleife 4 direkt die UV-Schutzschicht 12 angeordnet werden kann. Ist das Objekt 8 anstelle eines Beton- oder Stahlbauwerks beispielsweise eine Lkw-Plane, können sowohl die Ausgleichsschicht 10 als auch die Dioden 58 und die Abstandsschicht 11 zwischen diesen entfallen, gegebenenfalls auch die UV-Schutzschicht 12, so dass als Trägerelement 2 insbesondere direkt die Lkw-Plane dienen und auf dieser die Leiterbahn aufgebracht werden kann. Gerade die mäandrierte Leiterbahn 3 kann vorzugsweise auf der Innenseite der Lkw-Plane direkt aufgedruckt werden. Dementsprechend entfällt auch jegliche Art einer zusätzlichen Verbindungseinrichtung zum Verbinden der mäandrierten Leiterbahn 3 mit der Lkw-Plane, da aufgrund der haftenden Eigenschaft des Druckmaterials auf der Oberfläche der Lkw-Plane bereits die gewünschte Verbindung zwischen der mäandrierten Leiterbahn 3 und der Lkw-Plane als Trägerelement 2 geschaffen werden kann.

Die UV-Schutzschicht 12 kann als UV-Schutzfolie, als Lackschicht, somit als Decklackschicht, oder als Schaumschicht ausgebildet werden. Als Schutzschicht kann sie den Einflüssen durch Witterung, Sonneneinstrahlung und starken Temperaturschwankungen optimal widerstehen, um die Risserkennungseinrichtung 1 gegen Verspröden und Altern zu schützen.

Die Auswerteeinheit 6 kann vollständig oder in Teilen auf die Trägerelementoberfläche 20 aufgedruckt werden, ggf. zusammen mit der zumindest einen Leiterbahn. Hierdurch ist es möglich, eine einfache und robuste Kontaktierung von Leiterbahnen und Auswerteeinheit vorzunehmen. Die Auswerteeinheit 6 kann ferner Elemente zur Selbstüberwachung enthalten, um zuverlässig einen vollständigen oder zumindest teilweisen Ausfall der Elektronikkomponenten der Ausfalleinheit 6 erkennen zu können. Zum Testen von Eingangsports der Auswerteeinheit 6, an denen die Leiterbahnen in diese hineingeführt sind (siehe Figuren 1 bis 3), können dort Signalleitungen mit klar definiertem Zustand angeschlossen werden, also dem Zustand leitend bzw. dem Zustand nicht-leitend. Die Auswerteinheit 6 kann ferner Algorithmen zur Signalüberwachung enthalten, um gelegentliche Kontaktunterbrechungen durch Verspröden der Leiterbahn oder Leiterbahnen von einem echten Bruch von diesen und dem Auftreten eines Risses 9 im Objekt 8 unterscheiden zu können. Gerade aufgrund von Schwingungen, denen das Objekt 8 ausgesetzt ist, wie ein Brückenbauwerk, das durch den darüber geführten Verkehr regelmäßigen oder auch unregelmäßigen Belastungen ausgesetzt ist, können die Leiterbahnen verspröden bzw. Mikro-Risse in diesen auftreten. Solche Versprödungen und Mikro-Risse können dementsprechend aufgrund der entsprechenden Algorithmen der Auswerteeinheit von einem echten Bruch einer Leiterbahn unterschieden werden.

Die in Figur 5 gezeigte Ausführungsvariante der Risserkennungseinrichtung 1 umfasst mehr Schichten als die in Figur 4 gezeigte. Ihre grundsätzliche Funktionalität entspricht jedoch ansonsten dieser. Auf dem Objekt 8 bzw. dessen Objektoberfläche 80 ist in der Ausführungsvariante nach Figur 5 die Ausgleichsschicht 10 angeordnet, die wiederum zum Ausglich von Unebenheiten auf der Objektoberfläche 80 dient. Die Ausgleichsschicht 10 kann auch hier als aushärtende Spachtelmasse vorgesehen sein bzw. aus einem Zwei-Komponenten-Material bestehen. Auch bei dieser Ausführungsvariante ist auf der Ausgleichsschicht 10 bzw. deren Ausgleichschichtoberfläche 100 das Trägerelement 2, beispielsweise in Ausgestaltung als Polycarbonat-Folienmaterial als Unterlage die darüber aufgefügten Leiterbahnen aufgebracht. Das Trägerelement 2 kann wiederum auf seiner auf der der Trägerelementoberfläche 20 gegenüberliegenden Trägerelementunterseite 21 aufgeraut bzw. mit einer Strukturoberfläche versehen werden, um ein besonders gutes Aufkleben des Trägerelements 20 auf der Ausgleichsschicht 10 zu ermöglichen.

Auf der Trägerelementoberfläche 20 ist die zumindest eine Leiterbahn oder sind Leiterbahnen aufgedruckt oder als Leitpaste aufgebracht. Bei der in Figur 5 gezeigten Ausführungsvariante ist beispielhaft die Schicht 13 als Leitpastenschicht aus Leitpaste ausgebildet. Sie kann jedoch auch z.B. aufgedruckte oder anderweitig aufgebrachte Leiterbahnen umfassen. Auf der Oberfläche 130 der Leitpastenschicht 13 ist ein weiteres Folienelement 14, das beispielsweise ebenfalls als Polycarbonat-Folienmaterial bestehen kann, angeordnet. Das Folienelement 14 dient als Isolierschicht gegenüber der nächsten Schicht, die wiederum mit Leiterbahnen versehen ist. Diese Leiterbahnen sind auf der Oberfläche 140 des Folienelements 14 aufgebracht. Die Isolierschicht bzw. das Folienelement 14 dient zum elektrischen Isolieren der Leitpastenschicht 13 gegenüber den Leiterbahnen in der darüber befindlichen Schicht 15. Alternativ zum Anordnen von Leiterbahnen in der Schicht 15 kann eine Leitpaste auf die Isolierschicht bzw. das Folienelement 14 aufgebracht werden. Insbesondere ist es möglich, die sich kreuzenden Leiterbahnen des Gitters 5, also der Sensorfläche, wie es in Figur 3 skizziert ist, in unterschiedlichen Schichten der Risserfassungseinrichtung 1 anzuordnen, somit den einen Teil der sich kreuzenden Leiterbahnen in der Schicht 13 und den andere Teil in der Schicht 15. Auch hierdurch kann ein Muster aus sich kreuzenden Leiterbahnen in unterschiedlichen Ebenen der Risserkennungseinrichtung 1 erzeugt werden.

Im Bereich der jeweiligen Kreuzungspunkte 57 der Leiterbahnen in den Schichten 13 und 15 sind wiederum Dioden 58 eingeklebt und mit den Leiterbahnen mittels einer elektrisch leitfähigen Verbindungseinrichtung, wie beispielsweise einem elektrisch leitfähigen Kleber oder durch Löten, verbunden. Elektrische Kurzschlüsse können durch Lackieren der Oberfläche der mit den Leiterbahnen versehenen Schicht 15 außerhalb der Dioden 58 verhindert werden. In der in Figur 5 gezeigten Ausführungsvariante ist daher direkt auf die Oberfläche 150 der mit Leiterbahnen versehenen Schicht 15 und die Dioden 58 auf dieser eine elektrische Isolierschicht 16 aufgebracht. Diese kann beispielsweise als Lackschicht ausgebildet sein. Die Isolierschicht 16 dient der elektrischen Isolierung, ebenso wie die zuvor bereits erwähnte Isolierschicht 14.

Über der Isolierschicht 16, also auf deren Oberflächte 160, ist eine Schutzschicht 17 aufgebracht, die ebenfalls die Dioden 58 vor mechanischer Beschädigung schützen soll. Die Schutzschicht 17 kann beispielsweise aus Polyethylen bestehen. Auf der Schutzschichtoberfläche 170 ist die UV-Schutzschicht 12 aufgebracht. Die UV-Schutzschicht 12 dient auch hier gegen Verspröden bzw. Altern der darunterliegenden Schichten der Risserkennungseinrichtung 1. Beispielsweise kann die UV-Schutzschicht 12 als Polyurethan-Schaumschicht ausgebildet werden und als selbstklebende Schicht auf die Oberfläche 170 der Schutzschicht 17 aufgebracht werden.

Figur 6 zeigt eine gegenüber der Ausführungsvariante nach Figur 5 geringfügig modifizierte Ausführungsvariante der Risserkennungseinrichtung 1. Bei dieser ist um die Dioden 58 herum die Abstandsschicht 11 in Form z.B. eines Schaummaterials, wie eines Polyurethan-Schaummaterials, mit Aussparungen zum Platzieren der Dioden 58 auf der Schicht 15 mit den Leiterbahnen bzw. deren Oberfläche 150 aufgebacht. Die Dioden 58 sind ebenfalls auf der Oberfläche 150 der Leiterbahnen aufgefügt, z.B. aufgeklebt bzw. an diesen angelötet. Die Abstandsschicht 11 ist somit passepartoutartig um die einzelnen Dioden 58 herum angeordnet und kann verhindern, dass sich beim Transport und der Montage der Risserkennungseinrichtung 1 die Dioden von der Oberfläche 150 der Leiterbahnen ablösen.

Auf der Abstandsschicht 11 ist bei der Ausführungsform der Risserkennungseinrichtung 1 gemäß Figur 6 die Schutzschicht 17 aufgefügt, die die Dioden 58 zusätzlich vor mechanischer Beschädigung schützen soll. Zum mechanischen Schutz der Dioden 58 sind somit zwei Schichten bei der Ausführungsvariante nach Figur 6 vorgesehen, nämlich die Abstandsschicht 11 und die darüber aufgefügte Schutzschicht 17. Die Schutzschicht 17 kann beispielsweise als Polyethylen-Schutzfolie ausgebildet und auf die Abstandsschicht 11 aufgeklebt werden. Auf der Oberfläche 170 der Schutzschicht 17 ist bei der Ausführungsvariante der Risserkennungseinrichtung 1 gemäß Figur 6 die UV-Schutzschicht 12, die einen Schutz gegen UV-Strahlen ermöglicht, aufgebracht. Diese kann auch bei dieser Ausführungsvariante z.B. als selbstklebende Folie in Form einer Polyurethan-Schaumschicht ausgebildet und auf die Oberfläche 170 der Schutzschicht 17 aufgebracht werden. Die Schutzschicht 12 dient auch hier dazu, die darunterliegenden Schichten der Risserkennungseinrichtung 1 gegen Verspröden und Altern durch UV-Licht zu schützen.

Das jeweilige Trägerelement 2 kann beispielsweise Abmessungen von 3,0 m x 15 m bzw. Abmessungen von 0,4 m x 0,5 m bis 0,5 m x 1,5 m bzw. jegliche andere Abmessungen aufweisen, je nach Anwendungsfall bzw. je nach Objekt 8, das auf eventuelle Rissbildung oder Rissvergrößerung eines bestehenden Risses auf seiner Objektoberfläche untersucht bzw. überwacht werden soll. Wenn das Trägerelement 2 in Form einer Lkw-Plane ausgebildet ist, kann die zumindest eine mäanderförmig ausgebildete Leiterbahn 3 sich auch über die gesamte Lkw-Plane hinweg erstrecken, so dass die Lkw-Plane als Trägerelement die entsprechend gängigen Abmessungen einer solchen Lkw-Plane aufweist.

Die Ansprechschwelle der Risserkennungseinrichtung 1 kann so ausgelegt werden, dass sie Risse 9 ab einer Breite von 0,1 mm, insbesondere ab einer Breite von 0,2 mm, verlässlich erkennt. Ebenfalls ist es möglich, ein Risswachstum in einer entsprechenden Größenordnung mit der Risserkennungseinrichtung 1 verlässlich zu erkennen. Gerade bei Verwendung der Risserkennungseinrichtung 1 an einem Bauwerk, wie einem Brückenbauwerk, einer Fahrzeugrampe, einer Stellfläche in einem Parkhaus oder einem sonstigen Bauwerk, das auf einer Seite mechanisch belastet wird, wird die Risserkennungseinrichtung vorteilhaft auf der gegenüberliegenden, also der nicht mechanisch belasteten Seite des Bauwerk angeordnet, wie beispielsweise bei einer Brücke an deren Unterseite. Um ein Beschädigen der Lkw-Plane gerade durch Aufschlitzen verlässlich erkennen zu können, wird die Risserkennungseinrichtung bei Anordnen an einer Lkw-Plane an deren Innenseite, also der Seite, die zur Abdeckung der Ladefläche in Richtung der Ladefläche gerichtet wird, angeordnet. Dementsprechend ist die Risserkennungseinrichtung 1 von der Außenseite der Lkw-Plane aus für eventuelle Diebe nicht erkennbar. Die Risserkennungseinrichtung 1 kann gerade in der Anwendung an einer Lkw-Plane mit einer Einrichtung zum Ausgeben eines Alarms zusammenwirken oder versehen sein. Dementsprechend kann ein solcher Alarm bei Erkennen eines Risses durch die vollautomatisch arbeitende Risserkennungseinrichtung vollautomatisch ausgegeben werden. Bei einem plötzlichen Anstieg des elektrischen Widerstandes, was bei Erkennen eines Risses aufgrund Bruchs zumindest einer Leiterbahn durch die Auswerteeinheit festgestellt wird, kann vollautomatisch ein Alarm bzw. ein Alarmsignal ausgelöst werden. Ein solcher Alarm bzw. ein solches Alarmsignal kann direkt einen akustischen/oder optischen Alarm auslösen oder aber lediglich oder zusätzlich an eine zentrale Überwachungseinheit des Lkws weitergegeben werden.

Soll eine besonders großflächige Überwachung erfolgen, können gerade bei Beton- und Stahlbauwerken kaskadierte Trägerelemente, also mehrere Trägerelemente, die kaskadiert angeordnet und abgefragt werden, vorgesehen werden. Es ist somit jederzeit durch die Risserkennungseinrichtung das Erfassen der jeweiligen Position des auftretenden Risses auf dem Objekt, insbesondere Bauwerk, sowie der Zeit des Auftretens des Risses und insbesondere bei Ausführen der Risserkennungseinrichtung mit den sich kreuzenden Leiterbahnen und an den Kreuzungspunkten angeordneten Dioden auch die genaue Lage des aufgetretenen Risses erfassbar. Die erfassten Messdaten können in Echtzeit an eine zentrale Einheit zur weiteren Auswertung und zum Ergreifen von weiteren Maßnahmen weitergeleitet werden.

Neben den im Vorstehenden beschriebenen und in den Figuren gezeigten Ausführungsvarianten von Risserkennungseinrichtungen können noch zahlreiche weitere gebildet werden, auch beliebige Kombinationen der vorstehend genannten Merkmale von diesen, wobei die jeweilige Risserkennungseinrichtung jeweils vollautomatisch die Rissbildung und/oder Rissvergrößerung eines bereits bestehenden Risses auf der Objektoberfläche des Objekts, das überwacht wird, erkennt. Hierzu weist sie zumindest ein Trägerelement, auf dem zumindest eine Leiterbahnschleife und/oder mäandrierte Leiterbahn und/oder sich kreuzende und gegeneinander isolierte Leiterbahnen mit an den Kreuzungspunkten der sich kreuzenden Leiterbahnen angeordneten und mit den Leiterbahnen elektrisch verbundene Dioden angeordnet sind, und zumindest einen Teil einer oder zumindest eine Auswerteeinheit auf, wobei die Auswerteeinheit mit den Enden der Leiterbahn bzw. den Leiterbahnen verbunden ist und kontinuierlich die zumindest eine Leiterbahn auf Unterbrechungen überprüft. Das Trägerelement kann ggf. auch das Objekt selbst sein, wenn es sich bei dem Objekt beispielsweise um eine Lkw-Plane handelt.

### Bezugszeichenliste

- 1: Risserkennungseinrichtung
- 2: Trägerelement
- 3: mäandrierte Leiterbahn
- 4: Leiterbahnschleife
- 5: Gitter/Sensorfläche
- 6: Auswerteeinheit
- 7: Übertragungseinheit
- 8: Objekt
- 9: Riss
- 10: Ausgleichsschicht
- 11: Abstandsschicht
- 12: UV-Schutzschicht
- 13: Leitpastenschicht
- 14: Folienelement/Isolierschicht
- 15: Schicht mit Leiterbahnen
- 16: elektrische Isolierschicht
- 17: Schutzschicht
- 20: Trägerelementoberfläche
- 21: Trägerelementunterseite
- 30: erstes Leiterbahnende
- 31: zweites Leiterbahnende
- 40: erstes Leiterbahnschleifenende
- 41: zweites Leiterbahnschleifenende
- 50: erste Leiterbahn
- 50a: erstes Leiterbahnende
- 50b: zweites Leiterbahnende
- 51: zweiter Leiterbahn
- 51a: erstes Leiterbahnende
- 51b: zweites Leiterbahnende
- 52: dritte Leiterbahn
- 52a: erstes Leiterbahnende
- 52b: zweites Leiterbahnende
- 53: vierte Leiterbahn
- 53a: erstes Leiterbahnende
- 53b: zweites Leiterbahnende
- 54: Leiterbahn
- 55: fünfte Leiterbahn
- 55a: erstes Leiterbahnende
- 55b: zweites Leiterbahnende
- 56: sechste Leiterbahn
- 56a: erstes Leiterbahnende
- 56b: zweites Leiterbahnende
- 57: Kreuzungspunkt
- 58: Diode
- 59: Leiterbahn
- 80: Objektoberfläche
- 100: Ausgleichsschichtoberfläche
- 110: Abstandsschichtoberfläche
- 130: Oberfläche von 13
- 140: Oberfläche von 14
- 150: Oberfläche Leiterbahn
- 160: Oberfläche von 16
- 170: Oberfläche von 17

## Patentansprüche

1. Risserkennungseinrichtung (1) zur Überwachung eines Objekts (8) auf Rissbildung an der Objektoberfläche (80),
**dadurch gekennzeichnet, dass**
die Risserkennungseinrichtung (1) vollautomatisch die Rissbildung auf der Objektoberfläche (80) des Objekts (8) überwacht und zumindest ein Trägerelement (2) mit zumindest einer Leiterbahnschleife (3) und/oder zumindest einer mäandrierten Leiterbahn (4) und/oder sich kreuzenden und gegeneinander isolierten Leiterbahnen (50, 51, 52, 53, 54, 55, 56, 59) mit an den Kreuzungspunkten (57) der sich kreuzenden und gegeneinander isolierten Leiterbahnen (50, 51, 52, 53, 54, 55, 56, 59) angeordneten und mit den Leiterbahnen (50, 51, 52, 53, 54, 55, 56, 59) verbundenen Dioden (58), zumindest eine Verbindungseinrichtung zum Verbinden des zumindest einen Trägerelements (2) mit der Objektoberfläche (80) und zumindest eine oder zumindest einen Teil einer Auswerteeinheit (6) umfasst, wobei die zumindest eine Auswerteeinheit (6) mit den Enden (30, 31, 40, 41, 50a, 50b, 51 a, 51b, 52a, 52b, 53a, 53b, 55a, 55b, 56a, 56b) der Leiterbahn (3, 4) oder Leiterbahnen (50, 51, 52, 53, 54, 55, 56, 59) verbunden ist und kontinuierlich die Leiterbahn(en) (3, 4, 50, 51, 52, 53, 54, 55, 56, 59) auf Unterbrechungen überprüft.

2. Risserkennungseinrichtung (1) nach Anspruch 1,
**dadurch gekennzeichnet, dass**
die Risserkennungseinrichtung (1) zumindest eine Übertragungseinheit (7) zum Übertragen der von der zumindest einen Auswerteeinheit (6) ermittelten Daten zur Rissbildung an eine zentrale Einheit umfasst.

3. Risserkennungseinrichtung (1) nach Anspruch 1 oder 2,
**dadurch gekennzeichnet, dass**
zumindest eine Ausgleichsschicht (10) zum Ausgleich von Unebenheiten der Objektoberfläche (80) vorgesehen ist, insbesondere die zumindest eine Ausgleichsschicht (10) mit der zumindest einen Verbindungseinrichtung versehen ist.

4. Risserkennungseinrichtung (1) nach Anspruch 3,
**dadurch gekennzeichnet, dass**
die zumindest eine Ausgleichsschicht (10) zumindest ein Schaummaterial mit haftender oder klebender Oberfläche umfasst oder aus einem solchen besteht.

5. Risserkennungseinrichtung (1) nach einem der Ansprüche 1 bis 4,
**dadurch gekennzeichnet, dass**
die zumindest eine Ausgleichsschicht (10) aus zumindest einem Zwei-Komponenten-Material, insbesondere aus zumindest einem Zwei-Komponenten-Epoxidharz-Kleber oder einer aushärtenden Spachtelmasse, besteht.

6. Risserkennungseinrichtung (1) nach einem der vorstehenden Ansprüche, **dadurch gekennzeichnet, dass**
zum Schutz der an Kreuzungspunkten (57) von sich kreuzenden und gegeneinander isolierten Leiterbahnen (50, 51, 52, 53, 54, 55, 56, 59) angeordneten Dioden (58) vor mechanischer Beschädigung zumindest eine Abstandsschicht (11) zwischen den Dioden (58) angeordnet ist, deren Höhe zumindest der der Dioden (58) entspricht.

7. Risserkennungseinrichtung () nach einem der vorstehenden Ansprüche, **dadurch gekennzeichnet, dass**
zumindest eine für UV-Strahlen zumindest wenig durchlässige UV-Schutzschicht (12) vorgesehen und auf der der Witterung ausgesetzten Außenseite der Risserkennungseinrichtung (1) angeordnet ist.

8. Risserkennungseinrichtung (1) nach einem der vorstehenden Ansprüche, **dadurch gekennzeichnet, dass**
das zumindest eine Trägerelement (2) eine Kunststofffolie ist, insbesondere aus Polycarbonat besteht, und/oder das zumindest eine Trägerelement (2) Abmessungen von bis 3,0 m x 15 m aufweist, insbesondere Abmessungen von 0,4 m x 0,5 m bis 0,5 m x 1,5 m.

9. Risserkennungseinrichtung (1) nach einem der vorstehenden Ansprüche, **dadurch gekennzeichnet, dass**
die zumindest eine Leiterbahn (3, 4, 50, 51, 52, 53, 54, 55, 56, 59) auf das zumindest eine Trägerelement (2) aufgedruckt ist.

10. Risserkennungseinrichtung (1) nach einem der vorstehenden Ansprüche, **dadurch gekennzeichnet, dass**
das zumindest eine Trägerelement (2) einseitig aufgeraut ist, um eine gute Haftung der Ausgleichsschicht (10) auf der Trägerelementunterseite (21) zu ermöglichen.

11. Risserkennungseinrichtung (1) nach einem der vorstehenden Ansprüche, **dadurch gekennzeichnet, dass**
die Ansprechschwelle der Risserkennungseinrichtung (1) so ausgelegt ist, dass sie Risse (9) ab einer Breite von 0,1 mm, insbesondere ab einer Breite von 0,2 mm, und/oder ein Risswachstum in einer Größenordnung von zumindest 0,1 mm, insbesondere zumindest 0,2 mm, verlässlich erkennt.

12. Objekt (8) mit zumindest einer Risserkennungseinrichtung (1) nach einem der vorstehenden Ansprüche,
**dadurch gekennzeichnet, dass**
das Objekt (8) ein Bauwerk ist, insbesondere ein Beton- und/oder Stahlbauwerk, wie eine Brücke, eine Fahrzeugrampe, eine tragende Wand, eine Stellfläche in einem Parkhaus oder ein anderes tragendes Bauwerkselement, auf dessen Außenseite die Risserkennungseinrichtung aufgebracht ist, insbesondere die zumindest eine Risserkennungseinrichtung (1) an einer Seite oder Unterseite des Bauwerks angeordnet ist, die nicht mechanisch belastet wird..

13. Objekt (8) mit zumindest einer Risserkennungseinrichtung (1) nach einem der Ansprüche 1 bis 11,
**dadurch gekennzeichnet, dass**
das Objekt (8) eine Lkw-Plane eines Lastkraftwagens zur Abdeckung der Ladefläche des Lastkraftwagens ist, wobei die Risserkennungseinrichtung (1) zum Erkennen einer Beschädigung der Plane dient, die Lkw-Plane eine Innenseite und eine Außenseite aufweist und die zumindest eine Risserkennungseinrichtung (1) auf der Innenseite der Lkw-Plane angeordnet ist.

14. Objekt (8) nach Anspruch 13,
**dadurch gekennzeichnet, dass**
die Lkw-Plane als Trägerelement (2) der Risserkennungseinrichtung (1) ausgebildet und mit zumindest einer Leiterbahn (3) versehen ist und/oder ein zusätzliches Trägerelement (2) der Risserkennungseinrichtung (1) vorgesehen und mit zumindest einer Leiterbahn (3) versehen ist.

15. Objekt (8) nach Anspruch 13 oder 14,
**dadurch gekennzeichnet, dass**
die Risserkennungseinrichtung (1) mit zumindest einer Einrichtung zum Ausgeben eines Alarms zusammenwirkt zum Ausgeben eines Alarms bei Erkennen einer Beschädigung der Plane durch Aufschlitzen.
